# EUROPEAN PATENT APPLICATION

(11) **EP 1 655 015 A1**
(43) Date of publication of application: **10.05.2006**
(21) Application number: 03818083.2
(22) Date of filing: 11.08.2003
(51) Int. Cl.: A61K 8/18

(54) **HAIR REGENERATOR**

(71) Applicant: Franco Velasco, Andrés, 18151 Granada (ES)
(72) Inventor: Franco Velasco, Andrés, 18151 Granada (ES)
(74) Representative: Toro Gordillo, Ignacio Maria
(86) International application number: PCT/ES2003/000419
(87) International publication number: WO 2005/016297

(57) **Abstract**

The invention relates to a hair regenerator which is based on a regenerator such as that described in patent of invention 200200105, comprising a mixture of an epidermal growth factor, dexpanthenol and sodium hyaluronate, said mixture being applied by means of intradermic trichotherapy alternated with a surface cosmetic treatment. The invention consists in replacing the epidermal growth factor in the aforementioned patent, specifically a protein obtained through the genetic transformation of *Saccharomyces Cerevisae* yeast cells with DNA segment encoding the synthesis of said protein, with a protein comprising an embryonic amniotic liquid extract from cattle in the fifth week of gestation. In this way, the inventive regenerator provides a cure for existing pathologies relating to the scalp and produces capillary neoformation in a shorter amount of time and with higher quality indices.

## Description

### OBJECT OF THE INVENTION

The present invention refers to a hair regenerator intended for treating alopecic areas, a regenerator that can be indistinctly applied to either sex, by means of a minimally invasive technique, specifically by means of intradermal trichotherapy.

The object of the invention is to achieve a regenerator in which a protein-based epidermal growth factor participates, whereby managing to considerably improve skin quality and, as a result thereof, the quality and amount of already existing hair and the likely neoformation thereof, reducing treatment time and with higher quality ratings.

### BACKGROUND OF THE INVENTION

The same applicant is the owner of Spanish patent of invention with application number 200200105, which discloses a hair regenerator and the method of application thereof.

Said hair regenerator consists of a mixture of three different products, specifically the following:
- Epidermal growth factor.
- Dexpanthenol.
- Sodium hyaluronate.

More specifically, the epidermal growth factor consists of a protein obtained by genetically transforming *Saccharomyces cerevisiae* yeast cells with a DNA segment encoding the synthesis of this protein, participating in an amount of 2,000 nanograms per dose, whereas dexpanthenol, consisting of an alcohol corresponding to pantothenic acid, participates in an amount of 250 mg per dose, and sodium hyaluronate in an amount of 0.20 cm³ per dose.

The method of application of the regenerator thus obtained consists of intradermal trichotherapy of the components of said regenerator, with analgosedation of the patient, and by means of intradermal injection in the vascular area of the dermis. The first four sessions must be given weekly, whereas the following four sessions must be given every two weeks, and as a complement to both, specifically three days after each of them, cosmetic treatment must be applied to the patient, specifically an application of a biomembrane growth factor gel by means of mesophoresis.

### DESCRIPTION OF THE INVENTION

The hair regenerator proposed by the invention, starting from the same epidermal growth factor, dexpanthenol and sodium hyaluronate combination or mixture as in the previously mentioned patent and the same method of application thereof, focuses its features on the fact that this invention uses a protein obtained by concentrating an amniotic-embryonic fluid extract from bovids in the fifth week of gestation as an epidermal growth factor rather than the mentioned protein.

The concentration of the new protein used in the formula is now 300 nanograms/ml, both when said protein is used in the injectable form in the intradermal trichotherapy sessions and when it is used in the cosmetic treatment accompanying each one of them, whereas the rest of the composition, i.e. dexpanthenol and sodium hyaluronate, maintain the proportions of the earlier patent.

The same results are obtained with the new hair regenerator as with the regenerator of the earlier patent, but with a very significant reduction of the response times when it is used as an injectable form, while at the same time observing a very clear improvement in the quality of both the skin and the hair, when surface cosmetic treatment is carried out.

### PREFERRED EMBODIMENT OF THE INVENTION

According to the essential nature of the invention, a protein obtained by concentrating amniotic-embryonic fluid from bovids in the fifth week of gestation was used as the epidermal growth factor (E.G.F.), as previously mentioned, with the following features:
- State fluid.
- Color transparent/light brown.
- Odor typical.
- pH 5.8.
- Proteins 12.26 gr/l.
- No. of anaerobic germs < 10 germs/ml.

In any case the pH must be maintained between 5.5 and 6.5, and the proteins must in turn be comprised between 10 and 20 gr/1.

After concentrating this product for the use of the protein in the formula, said concentration is at about 300 nanograms/ml, which is mixed with the dexpanthenol at an amount of 250 mg and with sodium hyaluronate at an amount of 0.20 cm³, all per dose.

The same results are observed in the biopsies but with a very significant reduction in the response times with respect to the earlier patent.

In the tests carried out with surface treatment, that is, without the injectable form, a very clear improvement of both the quality of the skin (any prior disease heals), the quality and quantity of the already existing hair, and the likely hair neoformation are clinically observed.

In any case and as previously stated also, the neoformation and healing of prior pathologies of the scalp are achieved in less time and with higher quality ratings.

## Claims

1. A hair regenerator which, being indistinctly applicable in alopecic areas in both sexes and being materialized in a mixture of epidermal growth factor, dexpanthenol and sodium hyaluronate, is **characterized in that** said epidermal growth factor consists of an amniotic-embryonic fluid extract from bovids during the fifth week of gestation.

2. A hair regenerator according to claim 1, **characterized in that** said epidermal growth factor, that is, the amniotic-embryonic fluid extract, is concentrated at a magnitude of 300 nanograms/ml, both in the injectable regenerator and in the cosmetic regenerator for surface treatment, whereas dexpanthenol participates at an amount of 250 mg and sodium hyaluronate at an amount of 0.20 cm³, all per application dose of the regenerator.
